(19) Europäisches Patentamt / European Patent Office / Office européen des brevets

(11) **EP 4 074 316 A1**

## (12) EUROPEAN PATENT APPLICATION
published in accordance with Art. 153(4) EPC

(43) Date of publication:
**19.10.2022 Bulletin 2022/42**

(21) Application number: **20899362.6**

(22) Date of filing: **14.12.2020**

(51) International Patent Classification (IPC):
*A61K 31/575* (2006.01)    *A61K 31/4184* (2006.01)
*A61K 31/4985* (2006.01)    *A61P 35/00* (2006.01)
*A23L 33/10* (2016.01)

(52) Cooperative Patent Classification (CPC):
**A23L 33/10; A61K 31/4184; A61K 31/4985;
A61K 31/575; A61P 35/00**

(86) International application number:
**PCT/KR2020/018271**

(87) International publication number:
**WO 2021/118324 (17.06.2021 Gazette 2021/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.12.2019  KR 20190166966**

(71) Applicant: **Seoul National University R & DB
Foundation
Seoul 08826 (KR)**

(72) Inventor: **YEON, Seong Chan
Seoul 08741 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

## (54) PHARMACEUTICAL COMPOSITION FOR PREVENTING OR TREATING CANCER

(57) The present invention provides a pharmaceutical composition for preventing or treating cancer, which includes UCDA and a benzimidazole-based compound, the composition being expected to have an excellent anticancer effect as a mixture and having the benefit of having less side effects as cell toxicity is lower compared to anticancer drugs used in existing treatments.

EP 4 074 316 A1

[FIG. 7]

Combination 1: Fen 25%, TUD 25%, Vit E Succ 25%, Omega 25%
Combination 2: Fen 50%, TUD 20%, Vit E Succ 20%, Omega 10%

**Description**

[Technical Field]

The present invention relates to a pharmaceutical composition for preventing or treating cancer.

[Background Art]

Despite numerous studies being conducted, cancer is a significant problem worldwide and its conquest still remains as a major challenge in medical science. Chemotherapy is one of the major methods in cancer treatment therapy, and generally refers to treatment of cancer using one or more anticancer drugs (chemotherapeutic agents).

Many anticancer drugs are characterized by suppressing mitosis, and inhibit cell proliferation by targeting rapidly dividable cancer cells. Some anticancer drugs stop cell division, and some anticancer drugs trigger apoptosis to kill cells. Apart from such traditional chemotherapy, many efforts are being made to provide a more targeted therapy.

Most of various anticancer drugs currently used for the treatment of cancer have a problem of high toxicity. Specifically, chemotherapy has lots of side effects such as renal toxicity, liver toxicity, severe nausea and vomiting, bone marrow suppression, hair loss, cytopenia and the like. Therefore, it is urgently necessary to develop a new anticancer drug with high anticancer effects and without being harmful to the human body.

[Summary of Invention]

[Problems to be Solved by Invention]

It is an object of the present invention to provide a pharmaceutical composition for preventing or treating cancer, which includes ursodeoxycholic acid (UDCA) and a benzimidazole-based compound.

[Means for Solving Problems]

1. A pharmaceutical composition for preventing or treating cancer, the pharmaceutical composition including ursodeoxycholic acid (UDCA) and a benzimidazole-based compound.
2. The pharmaceutical composition according to the above 1, wherein the UDCA is at least one selected from the group consisting of UDCA, TUDCA and GUDCA.
3. The pharmaceutical composition according to the above 1, wherein the benzimidazole-based compound is at least one selected from the group consisting of fenbendazole, albendazole, mebendazole and flubendazole.
4. The pharmaceutical composition according to the above 1, wherein the UDCA and the benzimidazole-based compound are included in a molar ratio of 1: 0.5 to 5.
5. The pharmaceutical composition according to the above 1, wherein the UDCA is TUDCA, and the benzimidazole-based compound is fenbendazole.
6. The pharmaceutical composition according to the 1 above, further comprising at least one selected from the group consisting of vitamin E tocopherol, vitamin E succinate and omega 3.
7. The pharmaceutical composition according to the above 1, further comprising vitamin E succinate and omega 3.
8. The pharmaceutical composition according to the above 7, wherein the UDCA, benzimidazole-based compound, vitamin E succinate and omega 3 are included in a molar ratio of 1: 0.5 to 5: 0.5 to 3: 0.1 to 2.
9. The pharmaceutical composition according to the above 1, wherein the cancer is at least one selected from the group consisting of liver cancer, testicular cancer, glioblastoma, oral cancer, basal cell cancer, brain tumor, gallbladder cancer, biliary tract cancer, colorectal cancer, laryngeal cancer, retinocytoma, ampulla of Vater cancer, bladder cancer, peritoneal cancer, adrenal cancer, non-small cell lung cancer, tongue cancer, small cell lung cancer, small intestine cancer, meningioma, esophageal cancer, renal pelvic ureter cancer, renal cancer, malignant bone tumor, malignant soft tissue tumor, malignant pimple tumor, malignant melanoma, eye tumor, urethral cancer, gastric cancer, breast cancer, sarcoma, pharyngeal cancer, cervical cancer, endometrial cancer, uterine sarcoma, prostate cancer, metastatic brain tumor, rectal cancer, vaginal cancer, spinal cord tumor, salivary gland cancer, tonsil cancer, squamous cell cancer, lung cancer and anal cancer.
10. The pharmaceutical composition according to the above 1, wherein the cancer is liver cancer or cervical cancer.

[Advantageous effects]

The present invention provides a pharmaceutical composition for preventing or treating cancer, including ursodeoxycholic acid (UDCA) and a benzimidazole-based compound. Compared to therapeutics used as the existing anticancer drugs, the composition of the present invention has advantages of more excellent anticancer effects and less side effects.

[Brief Description of Drawings]

FIG. 1 is a flowchart schematically illustrating MTT assay process.
FIG. 2 illustrates confirmation of anticancer effects on cervical cancer by comparing the cell viability of HeLa cells in each treatment group.
FIG. 3 illustrates confirmation of anticancer effects on liver cancer by comparing the cell viability of Hepa cells and expressing the same as a percentage in each treatment group.
FIG. 4 illustrates the collected results of FIG. 3 as a single straight line graph.
FIG. 5 illustrates confirmation of anticancer effects on liver cancer by comparing the cell viability of Hepa cells and expressing the same as a percentage in each treatment group.
FIG. 6 illustrates confirmation of anticancer effects on liver cancer by comparing the cell viability of Hepa cells and expressing the same as a percentage in each treatment group.
FIG. 7 illustrates confirmation of anticancer effects on liver cancer by counting the number of living cells among Hepa cells in each treatment group.
FIG. 8 illustrates the collected results of FIG. 7 as a single straight line graph.
FIG. 9 illustrates confirmation of pH3 protein through Western blot analysis.

[Mode for Carrying out Invention]

Hereinafter, the present invention will be described in detail.
The present invention relates to a pharmaceutical composition for preventing or treating cancer, which includes ursodeoxycholic acid (UDCA) and a benzimidazole-based compound.
UDCA is an abbreviation of ursodeoxycholic acid, and UDCA according to the present invention includes all of UDCA, tauroursodeoxycholic acid (TUDCA) in which UDCA is conjugated with taurine, and glycoursodeoxycholic acid (GUDCA) in which UDCA is conjugated with glycine.
In the present invention, the UDCA, TUDCA or GUDCA may be a synthetic product or may be isolated and identified from the nutria gallbladder, and may include a component recombined after extraction of a water-soluble component, but it is not limited thereto.
With regard to extraction from the nutria gallbladder, specifically, it may be extracted from nutria bile, and the extraction method may use any extraction method known in the art without limitation thereof. For example, freeze-dried extraction, hot water extraction, extraction using lower alcohols having 1 to 4 carbon atoms, in the case of resin Z-802: CDCA, 99% extraction, HPLC extraction, ultrafiltration extraction, supercritical fluid chromatography extraction, capillary electrophoresis extraction method, or the like, may be used, but it is not limited thereto.
In the present invention, the benzimidazole-based compound may be, for example, fenbendazole, albendazole, mebendazole or flubendazole, and any benzimidazole-based compound used as an anthelmintic may be possible, but it is not limited thereto. The combination of UDCA and the benzimidazole-based compound may be variously selected, for example, all possible combinations of UDCA, GUDCA, TUDCA, and fenbendazole, albendazole, mebendazole and flubendazole exemplified above may be available. For example, TUDCA may be used as UDCA, and fenbendazole may be used as a benzimidazole-based compound.
A content ratio of UDCA and the benzimidazole-based compound is not particularly limited, and for example, UDCA and benzimidazole may be included in a molar ratio of 1: 0.5 to 5. For example, the molar ratio may be 1: 0.5 to 5, 1: 0.5 to 4, 1: 0.5 to 3, 1: 1 to 5, 1: 1 to 4, 1: 1 to 3, 1: 1 to 2.5, 1: 1.5 to 5, 1: 2 to 2.5, but it is not limited thereto. The above ratio may be appropriately adjusted depending on patients subjected to administration or results of clinical trial.
The pharmaceutical composition of the present invention may further include vitamin E tocopherol, vitamin E succinate or omega 3. In such a case, a size of tumor may be reduced to further improve anticancer efficacy.
The pharmaceutical composition of the present invention may further include at least one from the group consisting of vitamin E tocopherol, vitamin E succinate and omega 3, and for example, may further include vitamin E tocopherol, vitamin E succinate and omega 3, may further include vitamin E tocopherol and omega 3, and may further include vitamin E succinate and omega 3.

A mixing ratio of the above ingredients is not particularly limited, and for example, in a case of further including vitamin E succinate and omega, the molar ratio of UDCA, a benzimidazole-based compound, vitamin E succinate and omega 3 may be, for example, 1: 0.5 to 5 : 0.5 to 3: 0.1 to 2, 1: 0.5 to 3: 0.5 to 1.5: 0.2 to 1.5, 1: 0.8 to 2.8: 0.8 to 1.2: 0.4 to 1.2, but it is not limited thereto.

As the cancer to be prevented or treated in the present invention, any cancer may be applied without limitation thereof, and the cancer may include, for example, at least one selected from the group consisting of liver cancer, testicular cancer, glioblastoma, oral cancer, basal cell cancer, brain tumor, gallbladder cancer, biliary tract cancer, colorectal cancer, laryngeal cancer, retinocytoma, ampulla of Vater cancer, bladder cancer, peritoneal cancer, adrenal cancer, non-small cell lung cancer, tongue cancer, small cell lung cancer, small intestine cancer, meningioma, esophageal cancer, renal pelvic ureter cancer, renal cancer, malignant bone tumor, malignant soft tissue tumor, malignant pimple tumor, malignant melanoma, eye tumor, urethral cancer, gastric cancer, breast cancer, sarcoma, pharyngeal cancer, cervical cancer, endometrial cancer, uterine sarcoma, prostate cancer, metastatic brain tumor, rectal cancer, vaginal cancer, spinal cord tumor, salivary gland cancer, tonsil cancer, squamous cell cancer, lung cancer and anal cancer, and specifically liver cancer or cervical cancer, but it is not limited thereto.

In the present invention, the UDCA and the benzimidazole-based compound may be used by appropriately selecting a compound, a water-soluble extract, a recombinant thereof, etc. according to the desired formulation.

In the present invention, the pharmaceutical composition may further include a pharmaceutically acceptable carrier in addition to the above ingredients. The pharmaceutically acceptable carrier may include those commonly used, such as lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil, but it is not limited thereto. Further, the pharmaceutical composition of the present invention may include diluents or excipients such as fillers, extenders, binders, wetting agents, disintegrants, surfactants, and other pharmaceutically acceptable additives. The pharmaceutically acceptable additives may be included in an amount of 0.1 to 99.9 parts by weight ("wt. parts") based on the composition, but the amount thereof may be adjusted by clinical trials, and it is not limited thereto.

The pharmaceutical composition of the present invention may be administered orally or parenterally (e.g., application or intravenous, subcutaneous, or intraperitoneal injection), and for example, may be applied through oral administration or administration via injection, but it is not limited thereto.

UDCA, benzimidazole-based anthelmintics, etc. are used as oral agents, and the pharmaceutical composition of the present invention may be orally administered. Therefore, it is possible to administer and take the composition in a more convenient method compared to the conventional anticancer drugs that require an administration method such as intratumoral administration and vascular administration.

The pharmaceutical composition of the present invention has effects of protecting the liver because of including the UDCA-based compound, such that liver damage caused by taking the drug can be reduced while having less side effects thereof. Further, due to anticancer synergistic effects of the UDCA-based compound, the composition may exhibit superior effects than those of a pharmaceutical composition containing only the benzimidazole-based compound, and can be easily taken as an oral drug. Therefore, it is expected that the medication compliance may also be excellent.

Solid preparations for oral administration may include powders, granules, tablets, capsules, soft capsules, pills, and the like, but they are not limited thereto. Liquid formulations for oral use may include suspensions, solutions, emulsions, syrups, aerosols, etc. Other than water and liquid paraffin, which are commonly used as simple diluents, various excipients such as wetting agents, sweeteners, fragrances, and preservatives, etc. may be included, but they are not limited thereto. Preparations for parenteral administration may be formulated and used in the form of external preparations and sterile injection preparations such as sterilized aqueous solutions, solutions, non-aqueous solutions, suspensions, emulsions, eye drops, eye ointments, syrups, suppositories, aerosols, etc. according to the conventional methods. For example, the pharmaceutical composition may be produced and used in the form of creams, gels, patches, sprays, ointments, plasters, lotions, liniment agents, eye ointments, eye drops, pasta agents or cataplasma, but it is not limited thereto. A composition for topical administration may be anhydrous or aqueous depending on clinical prescription, and non-aqueous solvents and suspensions may include propylene glycol, polyethylene glycol, vegetable oils such as olive oil, and injectable esters such as ethyl oleate, but it is not limited thereto. As a base of the suppository, witepsol, macrogol, tween 61, cacao butter, laurin butter, glycerogelatin, etc. may be used, but it is not limited thereto.

A preferred dosage of the pharmaceutical composition of the present invention may vary depending on the absorption of the active ingredient in the body, the age, gender, and degree of obesity of the patient, but can be appropriately selected by those skilled in the art. However, to achieve the desired effects, in the case of oral administration, the composition of the present invention may be generally administered to an adult in an amount of 0.0001 to 500 mg/kg, and preferably, 0.001 to 500 mg/kg per kilogram of body weight per day. The composition may be administered once a day or in several divided doses. The above dosage and administration method do not limit the scope of the present invention in any way.

Hereinafter, examples will be described in detail to more concretely understand the present invention.

**Experimental method**

**1. Cell number counting method**

After culturing cells, the cells were resuspended in a fresh medium. After obtaining 200 μL of the cell suspension, 10 μL of trypsin blue with a dilution factor of 2× was added thereto.

A hemocytometer equipped with a coverslip was prepared, and each chamber was filled with the suspension, followed by counting the number of cells. A specific counting method is as follows.

```
     1) Live cells/ml = No. of cells counted / No. of squares

counted x Dilution factor x 1000

     2) Dead cells/ml = No. of dead cells / No. of squares

counted x Dilution factor x 100

     3) Percentage Viability = No. of live cells / Total No.

of cells x 100
```

**2. Cell Culture**

After establishing a sterile environment (clean bench, autoclave, 70% Ethanol, etc.), a cell growth medium (DMEM, FBS, Glutamine, Penicillin/Streptomycin) was prepared. Cells were identified and cultured under the above conditions.

**3. MTT assay**

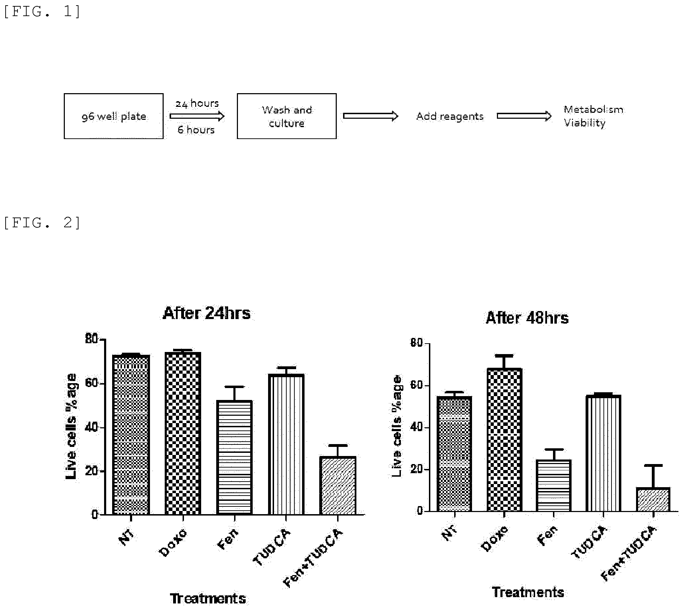

As illustrated in the flow chart shown in FIG. 1, the degree of cytotoxicity was evaluated through MTT assay.

**4. Western Blot Assessment**

Western blot assessment was performed to confirm a degree of production of pH3 protein in Hepa cells or HeLa cells treated with TUDCA or the like.

Total protein was obtained from the cells with a radioimmunoprecipitation assay buffer. The protein was digested in an 8 to 12% polyacrylamide gel and transferred to a nitrocellulose membrane. As a blocking buffer, 5% nonfat dry milk (1% Tween-20, in 20 mM TBS, pH 7.6) was used, and incubated with a primary antibody in the blocking buffer.

The primary antibody is an antibody obtained from Cell Signaling Technology in the USA or other countries, and a secondary antibody is anti-mouse or anti-rabbit IgG bound to HRP, and was obtained from GE Healthcare, Little Chalfont, UK.

Thereafter, the density of the band was measured by Western blot analysis.

**Results of experiment**

**1. Comparison of cell viability of HeLa cells**

(1) HeLa cells are cervical cancer cell lines. In this experiment, experimental groups were divided as follows: specifically, a group in which HeLa cells are not treated, that is, no treatment group (NT); a doxorubicin-treated group (Doxo); a fenbendazole-treated group (Fen); a TUDCA treated group (TUDCA); and a group treated with a mixture of fenbendazole and TUDCA (Fen+TUDCA), and cell viability was compared over time (24h, 48h) after treatment. Treatment was implemented using TUDCA at a concentration of 1000 μM, fenbendazole at a concentration of 1.2 μM, and doxorubicin at a concentration of 10 μM, respectively. Further, the mixture of fenbendazole and TUDCA was prepared by mixing both compounds with the above concentrations at the same dose. Further, all treatment groups were treated with the corresponding compounds at the same dose.

(2) As shown in FIG. 2, when compared to the effect of doxorubicin which is generally used as an anticancer drug, it could be seen that all of fenbendazole, TUDCA, the mixture of fenbendazole and TUDCA have more excellent apoptotic effects at 24h and 48h after treatment than doxorubicin, thereby expecting better anticancer effects. Further, in the case of the fenbendazole and TUDCA mixture treatment group, the effect was the most excellent over all times.

## 2. Comparison of cell viability of Hepa cells

(1) In the present invention, all liver cancer cell lines were Murin hepatoma cell lines (Hepalclc7), hereinafter, referred to as Hepa cells. In this experiment, the following definitions were applied: no treatment group (NT); a fenbendazole treatment group (Fen); a TUDCA 200 μmol treatment group (TUDCA 200); a TUDCA 500 μmol treatment group (TUDCA 500); a TUDCA 1000 μmol treatment group (TUDCA 1000); a mixture of 1.2 μmol fenbendazole and 200 μmol TUDCA (1.2+200); a mixture of 1.2 μmol fenbendazole and 500 μmol TUDCA (1.2+500); a mixture of 1.2 μmol fenbendazole and 1000 μmol TUDCA (1.2+1000), respectively. Each treatment group was treated with the corresponding compound at the concentration set to 100 nM. A mixing ratio in the mixture was 1:1 unless otherwise specified.

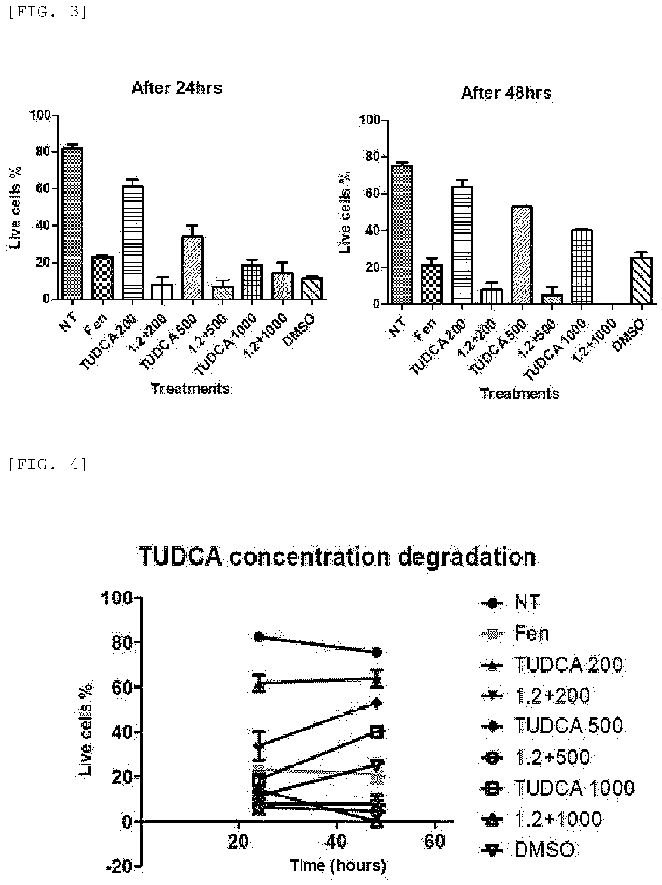

(2) As shown in FIG. 3, in the liver cancer cell lines, all treatment groups exhibited apoptosis effects compared to the NT group, and specifically, the group treated with a mixture of fenbendazole and TUDCA had the best effects. It could be seen that the higher the concentration of TUDCA, the better the apoptosis effect at 48 h, that is, the anticancer effects.

FIG. 4 illustrates the collected results of FIG. 3 as a dotted line graph, and the results are the same as shown in FIG. 3.

## 3. Comparison of Hepa cell viability in fenbendazole and UDCA compound treatment groups

(1) This experiment was performed to compare cell viability by treating liver cancer cell lines with each of the mixtures of fenbendazole and UDCA, fenbendazole and TUDCA, fenbendazole and GUDCA, respectively, while the control was treated with DMSO.

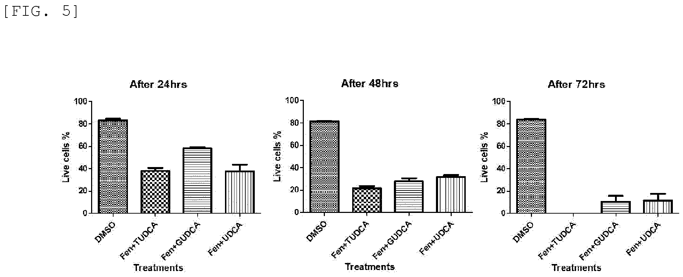

(2) As shown in FIG. 5, the apoptosis effect was excellent in all groups treated with the mixtures, and it was confirmed that the effect was the most excellent in the mixture of fenbendazole and TUDCA. Specifically, the cell viability was close to 0% at 72 h after treatment with the fenbendazole and TUDCA mixture. Table 1 below shows the results of this experiment as numerical values, and FIG. 5 shows the average values of the tables below.

[TABLE 1]

| | Total cells | Dead | Live | Live % | Total cells | Dead | Live | Live % | Total cells | Dead | Live | Live % |
|---|---|---|---|---|---|---|---|---|---|---|---|---|
| | | | | | | After 24hr | | | | | | |
| DMSO | 16 | 3 | 13 | 81.25 | 14 | 2 | 12 | 85.71429 | 17 | 3 | 14 | 82.35294 |
| Fen+TUDCA | 8 | 5 | 3 | 37.5 | 6 | 4 | 2 | 33.33333 | 7 | 4 | 3 | 42.85714 |
| Fen+GUDCA | 7 | 3 | 4 | 57.14286 | 5 | 2 | 3 | 60 | 7 | 3 | 4 | 57.14286 |
| Fen+UDCA | 9 | 5 | 4 | 44.44444 | 4 | 3 | 1 | 25 | 7 | 4 | 3 | 42.85714 |
| | | | | | | After 48hr | | | | | | |
| DMSO | 16 | 3 | 13 | 81.25 | 16 | 3 | 13 | 81.25 | 11 | 2 | 9 | 81.81818 |
| Fen+TUDCA | 5 | 4 | 1 | 20 | 5 | 4 | 1 | 20 | 4 | 3 | 1 | 25 |
| Fen+GUDCA | 4 | 3 | 1 | 25 | 4 | 3 | 1 | 25 | 6 | 4 | 2 | 33.33333 |
| Fen+UDCA | 3 | 2 | 1 | 33.33333 | 7 | 5 | 2 | 28.57143 | 6 | 4 | 2 | 33.33333 |
| | | | | | | After 72hr | | | | | | |
| DMSO | 37 | 6 | 31 | 83.78378 | 42 | 7 | 35 | 83.33333 | 26 | 4 | 22 | 84.61538 |
| Fen+TUDCA | 4 | 4 | 0 | 0 | 5 | 5 | 0 | 0 | 3 | 3 | 0 | 0 |
| Fen+GUDCA | 6 | 5 | 1 | 16.66667 | 7 | 6 | 1 | 14.28571 | 4 | 4 | 0 | 0 |
| Fen+UDCA | 7 | 6 | 1 | 14.28571 | 5 | 4 | 1 | 20 | 5 | 5 | 0 | 0 |

**4. Comparison of cell viability according to treatment of various mixtures**

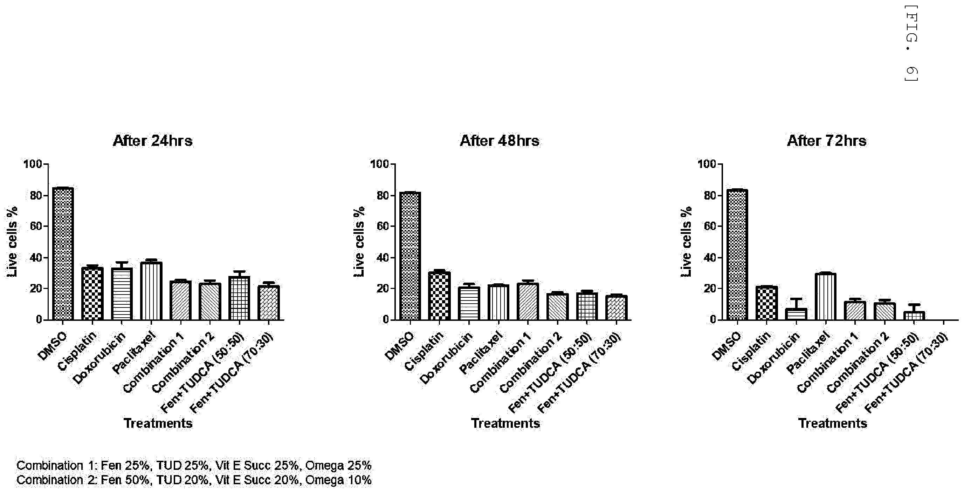

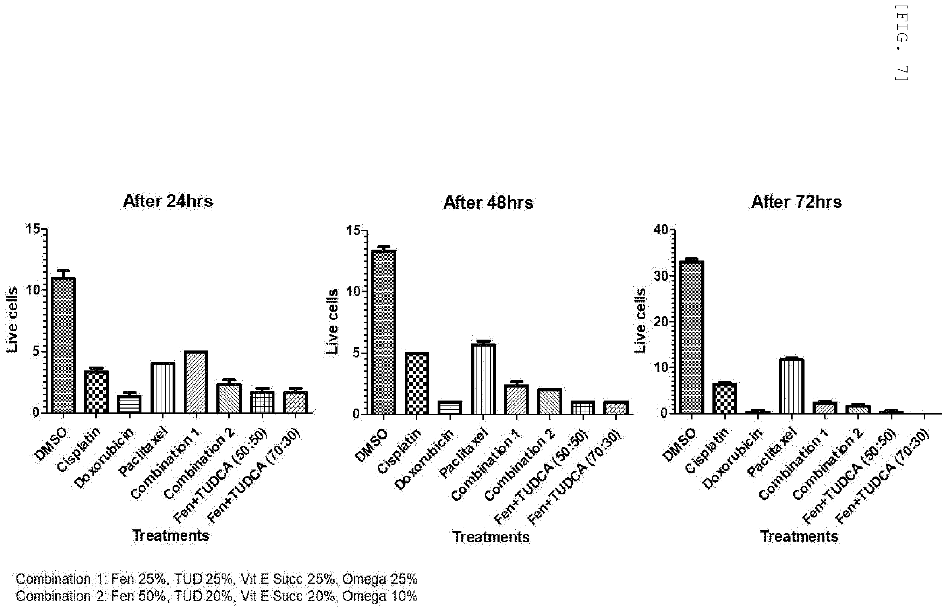

(1) This experiment was performed to confirm cell viability by treating liver cancer cell lines with various mixtures including cisplatin, doxorubicin, paclitaxel, and fenbendazole, which are used as conventional anticancer drugs.
(2) As shown in FIGS. 6 and 7, it could be confirmed from the results at 24h and 48h after treatment that the apoptosis effect in the group treated with the mixture containing fenbendazole was similar or superior to or than the conventional anticancer drug treatment group. This means that anticancer effects of the mixture containing fenbendazole are excellent. Further, in the case of 72h after treatment, it could be seen that the cell viability is close to 0% in the group treated with a 70:30 mixture of fenbendazole and TUDCA.

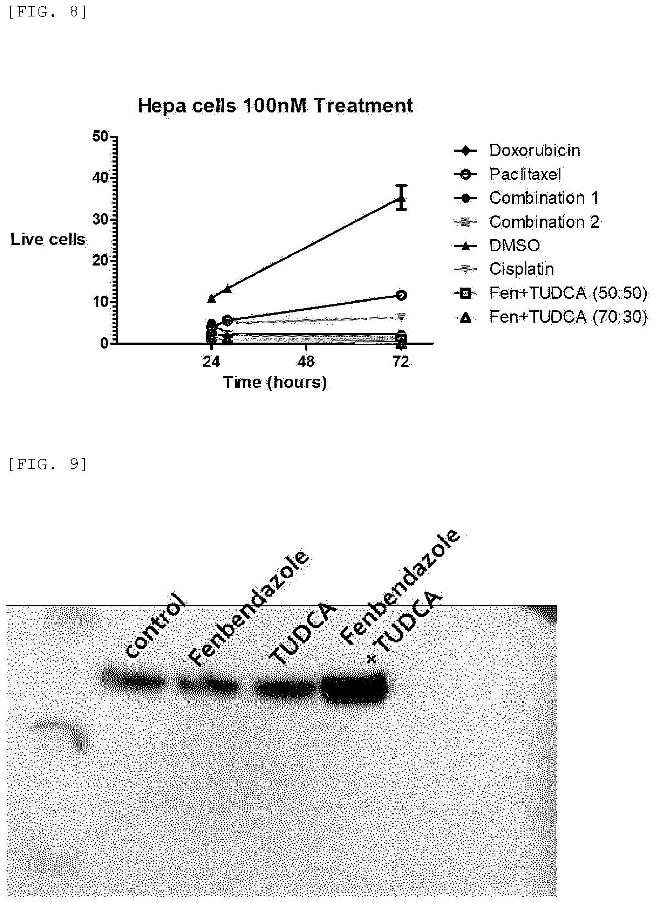

Further, it could be seen that the higher the mixing ratio of fenbendazole in the mixture of fenbendazole and TUDCA, the more excellent the anticancer effects. FIG. 8 shows the collected results of FIG. 7 as a single straight line graph, Table 2 below shows data of FIGS. 6 and 7 as numerical values, and FIGS. 6 and 7 show the average of the three experimental values as a graph.

[TABLE 2]

| | Total cells | Dead | Live | Live % | Total cells | Dead | Live | Live % | Total cells | Dead | Live | Live % |
|---|---|---|---|---|---|---|---|---|---|---|---|---|
| | | | | | After 24hrs | | | | | | | |
| DMSO | 12 | 2 | 10 | 83.33333 | 14 | 2 | 12 | 85.71429 | 13 | 2 | 11 | 84.61538 |
| Cisplatin | 9 | 6 | 3 | 33.33333 | 10 | 7 | 3 | 30 | 11 | 7 | 4 | 36.36364 |
| Paclitaxel | 11 | 7 | 4 | 36.36364 | 10 | 6 | 4 | 40 | 12 | 8 | 4 | 33.33333 |
| Doxorubicin | 3 | 2 | 1 | 33.33333 | 4 | 3 | 1 | 25 | 5 | 3 | 2 | 40 |
| Combination 1 | 21 | 16 | 5 | 23.80952 | 22 | 17 | 5 | 22.72727 | 19 | 14 | 5 | 26.31579 |
| Combination 2 | 10 | 8 | 2 | 20 | 9 | 7 | 2 | 22.22222 | 11 | 8 | 3 | 27.27273 |
| Fen+TUDCA (50:50) | 6 | 4 | 2 | 33.33333 | 5 | 4 | 1 | 20 | 7 | 5 | 2 | 28.57143 |
| Fen+TUDCA (70:30) | 9 | 7 | 2 | 22.22222 | 8 | 6 | 2 | 25 | 6 | 5 | 1 | 16.66667 |
| | | | | | After 48hrs | | | | | | | |
| DMSO | 17 | 3 | 14 | 82.35294 | 16 | 3 | 13 | 81.25 | 16 | 3 | 13 | 81.25 |
| Cisplatin | 17 | 12 | 5 | 29.41176 | 18 | 13 | 5 | 27.77778 | 15 | 10 | 5 | 33.33333 |
| Paclitaxel | 26 | 20 | 6 | 23.07692 | 28 | 22 | 6 | 21.42857 | 24 | 19 | 5 | 20.83333 |
| Doxorubicin | 4 | 3 | 1 | 25 | 6 | 5 | 1 | 16.66667 | 5 | 4 | 1 | 20 |
| Combination 1 | 11 | 8 | 3 | 27.27273 | 10 | 8 | 2 | 20 | 9 | 7 | 2 | 22.22222 |
| Combination 2 | 11 | 9 | 2 | 18.18182 | 8 | 6 | 2 | 25 | 8 | 6 | 2 | 25 |
| Fen+TUDCA (50:50) | 6 | 5 | 1 | 16.66667 | 7 | 6 | 1 | 14.28571 | 5 | 4 | 1 | 20 |
| Fen+TUDCA (70:30) | 7 | 6 | 1 | 14.28571 | 7 | 6 | 1 | 14.28571 | 6 | 5 | 1 | 16.66667 |
| | | | | | After 72hrs | | | | | | | |
| DMSO | 40 | 7 | 33 | 82.5 | 41 | 7 | 34 | 82.92683 | 38 | 6 | 32 | 84.21053 |
| Cisplatin | 30 | 24 | 6 | 20 | 32 | 25 | 7 | 21.875 | 29 | 23 | 6 | 20.68966 |
| Paclitaxel | 41 | 29 | 12 | 29.26829 | 36 | 25 | 11 | 30.55556 | 43 | 31 | 12 | 27.90698 |
| Doxorubicin | 3 | 3 | 0 | 0 | 3 | 3 | 0 | 0 | 5 | 4 | 1 | 20 |
| Combination 1 | 21 | 19 | 2 | 9.52381 | 20 | 17 | 3 | 15 | 21 | 19 | 2 | 9.52381 |
| Combination 2 | 18 | 17 | 1 | 5.555556 | 17 | 15 | 2 | 11.76471 | 15 | 13 | 2 | 13.33333 |
| Fen+TUDCA (50:50) | 4 | 4 | 0 | 0 | 7 | 6 | 1 | 14.28571 | 2 | 2 | 0 | 0 |
| Fen+TUDCA (70:30) | 3 | 3 | 0 | 0 | 2 | 2 | 0 | 0 | 3 | 3 | 0 | 0 |

### 5. Western Blot Assessment

(1) The pH3 protein confirmed in this experiment is a protein increasing when the cell cycle is interrupted, and appearance of a distinct band due to an increase in the pH3 protein means that the cell cycle is effectively stopped. (2) In the group treated with fenbendazole and TUDCA together, it could be seen that the pH3 protein was remarkably increased through the distinctly darkened band as a result of Weston blot, and this result means that the cell cycle of the cancer cell line was interrupted, thus indicating expression of anticancer effects. As shown in FIG. 9, it could be seen that the band was most intense and distinct in the fenbendazole and TUDCA mixture treatment group. Through this, it could be considered that the cell cycle of cancer cell lines is better interrupted when treated with the mixture of fenbendazole and TUDCA than the group treated with fenbendazole and TUDCA alone, thereby indicating better anticancer effects.

### Claims

1. A pharmaceutical composition for preventing or treating cancer, the pharmaceutical composition comprising ursodeoxycholic acid (UDCA) and a benzimidazole-based compound.

2. The pharmaceutical composition according to claim 1, wherein the UDCA is at least one selected from the group consisting of UDCA, TUDCA and GUDCA.

3. The pharmaceutical composition according to claim 1, wherein the benzimidazole-based compound is at least one selected from the group consisting of fenbendazole, albendazole, mebendazole and flubendazole.

4. The pharmaceutical composition according to claim 1, wherein the UDCA and the benzimidazole-based compound are included in a molar ratio of 1: 0.5 to 5.

5. The pharmaceutical composition according to claim 1, wherein the UDCA is TUDCA, and the benzimidazole-based compound is fenbendazole.

6. The pharmaceutical composition according to claim 1, further comprising at least one selected from the group consisting of vitamin E tocopherol, vitamin E succinate and omega 3.

7. The pharmaceutical composition according claim 1, further comprising vitamin E succinate and omega 3.

8. The pharmaceutical composition according to claim 7, wherein the UDCA, benzimidazole-based compound, vitamin E succinate and omega 3 are included in a molar ratio of 1: 0.5 to 5: 0.5 to 3: 0.1 to 2.

9. The pharmaceutical composition according to claim 1, wherein the cancer is at least one selected from the group consisting of liver cancer, testicular cancer, glioblastoma, oral cancer, basal cell cancer, brain tumor, gallbladder cancer, biliary tract cancer, colorectal cancer, laryngeal cancer, retinocytoma, ampulla of Vater cancer, bladder cancer, peritoneal cancer, adrenal cancer, non-small cell lung cancer, tongue cancer, small cell lung cancer, small intestine cancer, meningioma, esophageal cancer, renal pelvic ureter cancer, renal cancer, malignant bone tumor, malignant soft tissue tumor, malignant pimple tumor, malignant melanoma, eye tumor, urethral cancer, gastric cancer, breast cancer, sarcoma, pharyngeal cancer, cervical cancer, endometrial cancer, uterine sarcoma, prostate cancer, metastatic brain tumor, rectal cancer, vaginal cancer, spinal cord tumor, salivary gland cancer, tonsil cancer, squamous cell cancer, lung cancer and anal cancer.

10. The pharmaceutical composition according to claim 1, wherein the cancer is liver cancer or cervical cancer.

[FIG. 1]

| 96 well plate | 24 hours ⟹ 6 hours | Wash and culture | ⟹ | Add reagents | ⟹ | Metabolism Viability |

[FIG. 2]

[FIG. 3]

[FIG. 4]

[FIG. 5]

[FIG. 6]

After 24hrs

After 48hrs

After 72hrs

Combination 1: Fen 25%, TUD 25%, Vit E Succ 25%, Omega 25%
Combination 2: Fen 50%, TUD 20%, Vit E Succ 20%, Omega 10%

[FIG. 7]

After 24hrs

After 48hrs

After 72hrs

Combination 1: Fen 25%, TUD 25%, Vit E Succ 25%, Omega 25%
Combination 2: Fen 50%, TUD 20%, Vit E Succ 20%, Omega 10%

[FIG. 8]

**Hepa cells 100nM Treatment**

[FIG. 9]

| | |
|---|---|
| **INTERNATIONAL SEARCH REPORT** | International application No.<br>**PCT/KR2020/018271** |

**A. CLASSIFICATION OF SUBJECT MATTER**

**A61K 31/575**(2006.01)i; **A61K 31/4184**(2006.01)i; **A61K 31/4985**(2006.01)i; **A61P 35/00**(2006.01)i; **A23L 33/10**(2016.01)i

According to International Patent Classification (IPC) or to both national classification and IPC

**B. FIELDS SEARCHED**

Minimum documentation searched (classification system followed by classification symbols)

A61K 31/575(2006.01); A61K 31/20(2006.01); A61K 31/202(2006.01); A61K 38/02(2006.01); A61K 39/395(2006.01); A61K 47/30(2006.01); A61K 47/36(2006.01); A61P 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 암 (cancer), 우르소데옥시콜산 (UDCA, ursodeoxycholic acid), 벤즈이미다졸 (benzimidazole), 비타민 E 숙신산염 (vitamin E succinate), 오메가 3 (omega-3)

**C. DOCUMENTS CONSIDERED TO BE RELEVANT**

| Category* | Citation of document, with indication, where appropriate, of the relevant passages | Relevant to claim No. |
|---|---|---|
| A | US 2012-0082659 A1 (LAND, H. et al.) 05 April 2012 (2012-04-05)<br>See abstract; paragraph [0050]; and claims 1, 10, 11, 14, 17 and 18. | 1-10 |
| A | KR 10-2016-0117033 A (THE INDUSTRY & ACADEMIC COOPERATION IN CHUNGNAM NATIONAL UNIVERSITY (IAC)) 10 October 2016 (2016-10-10)<br>See entire document. | 1-10 |
| A | KR 10-2011-0088007 A (INDUSTRY-ACADEMIC COOPERATION FOUNDATION, CHOSUN UNIVERSITY) 03 August 2011 (2011-08-03)<br>See entire document. | 1-10 |
| A | YAM, D. et al. Suppression of tumor growth and metastasis by dietary fish oil combined with vitamins E and C and cisplatin. Cancer chemotherapy and pharmacology. 2001, vol. 47, pp. 34-40.<br>See entire document. | 1-10 |
| A | WO 2011-050302 A2 (UNIVERSITY OF SOUTHERN CALIFORNIA) 28 April 2011 (2011-04-28)<br>See entire document. | 1-10 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **22 March 2021** | **22 March 2021** |

| Name and mailing address of the ISA/KR | Authorized officer |
|---|---|
| Korean Intellectual Property Office<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

<table>
<tr><td colspan="2" align="center">**INTERNATIONAL SEARCH REPORT**<br>Information on patent family members</td><td colspan="2">International application No.<br>**PCT/KR2020/018271**</td></tr>
</table>

| Patent document cited in search report | | | Publication date (day/month/year) | Patent family member(s) | | | Publication date (day/month/year) |
|---|---|---|---|---|---|---|---|
| US | 2012-0082659 | A1 | 05 April 2012 | AU | 2008-307544 | A1 | 09 April 2009 |
| | | | | CA | 2700257 | A1 | 09 April 2009 |
| | | | | EP | 2188630 | A2 | 26 May 2010 |
| | | | | EP | 2665739 | A1 | 27 November 2013 |
| | | | | US | 2010-0285001 | A1 | 11 November 2010 |
| | | | | US | 2012-0114670 | A1 | 10 May 2012 |
| | | | | WO | 2009-045443 | A2 | 09 April 2009 |
| | | | | WO | 2009-045443 | A3 | 30 December 2009 |
| | | | | WO | 2012-100248 | A1 | 26 July 2012 |
| KR | 10-2016-0117033 | A | 10 October 2016 | KR | 10-1748127 | B1 | 16 June 2017 |
| KR | 10-2011-0088007 | A | 03 August 2011 | KR | 10-1131675 | B1 | 28 March 2012 |
| | | | | WO | 2011-093559 | A1 | 04 August 2011 |
| WO | 2011-050302 | A2 | 28 April 2011 | AU | 2010-310515 | A1 | 17 May 2012 |
| | | | | AU | 2010-310515 | B2 | 07 January 2016 |
| | | | | AU | 2016-201607 | A1 | 31 March 2016 |
| | | | | AU | 2016-201607 | B2 | 30 November 2017 |
| | | | | BR | 112012009423 | A2 | 13 June 2017 |
| | | | | CA | 2779282 | A1 | 28 April 2011 |
| | | | | CA | 2779282 | C | 05 December 2017 |
| | | | | CN | 102753162 | A | 24 October 2012 |
| | | | | DK | 2490684 | T3 | 09 April 2018 |
| | | | | EP | 2490684 | A2 | 29 August 2012 |
| | | | | EP | 2490684 | B1 | 03 January 2018 |
| | | | | ES | 2659353 | T3 | 14 March 2018 |
| | | | | JP | 2013-508411 | A | 07 March 2013 |
| | | | | PL | 2490684 | T3 | 31 July 2018 |
| | | | | PT | 2490684 | T | 02 April 2018 |
| | | | | RU | 2012118588 | A | 27 November 2013 |
| | | | | RU | 2549954 | C2 | 10 May 2015 |
| | | | | US | 2011-0118528 | A1 | 19 May 2011 |
| | | | | WO | 2011-050302 | A3 | 25 August 2011 |

Form PCT/ISA/210 (patent family annex) (July 2019)